# EUROPEAN PATENT APPLICATION

(11) **EP 1 170 373 A1**
(43) Date of publication of application: **09.01.2002**
(21) Application number: 00401899.0
(22) Date of filing: 03.07.2000
(51) Int. Cl.: C12N 15/87, A61K 48/00, A61P 27/02, A61K 38/10, A61K 38/18, A61K 47/42, A61K 47/48

(54) **Peptide-mediated ocular cell transfection**

(71) Applicant: Retina France, 31771 Colomiers Cedex (FR); UNIVERSITE RENE DESCARTES (PARIS V), F-75270 Paris Cédex 06 (FR)
(72) Inventor: Neuner-Jehle, Martin, 91100 Gif sur Yvette (FR); van den Berghe, Loic, 75015 Paris (FR); Bonnel, Sébastien, 75015 Paris (FR); Uteza, Yves, 94800 Villejuif (FR); Menasche, Maurice, 95400 Villiers le Bel (FR); Dufier, Jean-Louis, 75015 Paris (FR); Abitbol, Marc, 75015 Paris (FR)
(74) Representative: Becker, Philippe

(57) **Abstract**

This invention relates to compositions and methods for delivering a polynucleotide to ocular cells, in vitro, ex vivo or in vivo. More particularly, the invention resides, inter alia, in the use of a cationic peptide and a fusogenic peptide (or a combination thereof) in the preparation of a composition for delivering a polynucleotide to an ocular cell in vitro, ex vivo or in vivo. The invention can be used in many different areas such as experimental biology, preclinical and clinical research, as well as in therapeutic or prophylactic treatments of mammalians, in particular human subjects.

## Description

The instant invention relates to novel methods of transfecting ocular cells, in particular retinal cells, in vitro or in vivo. The invention also relates to compositions for use in said methods, and their use in the delivery of various polynucleotides to ocular cells. The invention can be used in many different areas such as experimental biology, preclinical and clinical research, as well as in therapeutic or prophylactic treatments of mammalians, in particular human subjects.

In recent years, significant progress has been made in developing protocols which allow an efficient transfer of therapeutically important genes to diseased tissues. These strategies require the mastering of techniques such as transplantation of genetically engineered cells, tissue infection by recombinant, replication-deficient viruses, or non-viral *in vivo* cell transfection. The last technique may be the safest therapeutic approach in terms of cell rejection or immune reaction.
Efficient *in vivo* cell transfection protocols are not yet available for delivering polynucleotides to ocular cells, more particularly suited for a potential treatment of age-related macular degeneration (AMD). Viral-mediated gene delivery to ocular cells has been disclosed with certain types of viral vectors, including adenoviral vectors or AAV vectors. In addition, the grafting of genetically modified cells to the ocular globe has also been reported. These methods, however, may involve certain drawbacks in terms of industrial scale-up and/or safety. There is therefore a need for alternative gene delivery methods, allowing efficient gene transfer and expression in ocular cells in vitro and in vivo, offering higher safety characteristics and easy scaling-up.

The invention now provides such compositions and methods which allow an improved polynucleotide delivery to ocular cells. Improved means for instance that the methods of the present invention provide increased safety and/or transfer efficiency and/or expression efficiency into ocular cells. More preferably, the invention resides in a novel method of peptide-mediated ocular gene delivery. In this respect, the invention now discloses that efficient and stable polynucleotide transfer and expression in ocular cells can be obtained when said polynucleotide is associated with a combination of synthetic peptides.

An object of the present invention therefore resides in a method of delivering a polynucleotide to an ocular cell in vitro, ex vivo or in vivo, comprising contacting said ocular cell with a composition comprising said polynucleotide complexed with a cationic peptide and a fusogenic peptide.
An object of the invention also lies in a method of delivering a polynucleotide to an ocular cell in vitro, ex vivo or in vivo, comprising:
(a) contacting said polynucleotide with a cationic peptide and a fusogenic peptide to form a complex between said polynucleotide and said peptides, and
(b) contacting the ocular cell with said complex.

The invention also resides in the use of a cationic peptide and a fusogenic peptide in the preparation of a composition for delivering a polynucleotide to an ocular cell in vitro, ex vivo or in vivo.

More preferably, the invention is used for delivering a polynucleotide to retinal cells. Even more preferably, the invention is used for delivering a polynucleotide to retinal pigment epithelial cells or choroidal cell.

The invention resides in the use of a combination of a cationic peptide and a fusogenic peptide to effect gene delivery to ocular cells. The invention shows that such a combination allows improved transfer and stable expression in ocular cells, in vitro and in vivo. The invention further shows that such a peptide combination allows preferential gene delivery to certain types of ocular cells, such as retinal pigment epithelium cells and choroidal cells. This is particularly advantageous since many ocular pathologies such as retinal pathologies result from alteration(s) of said cells.

Both the cationic and fusogenic peptides are preferably synthetic peptides, which can be prepared according to various conventional techniques, including peptide synthesis.

In a particular embodiment, the cationic peptide comprises 3 to 60 amino acid residues, preferably 5 to 40, more preferably 5 to 20. The peptide is "cationic" in that it comprises a positive net charge and can associate with nucleic acids, generally leading to nucleic acid condensation. The cationic peptides thus generally comprise amino acid residues with ε-amino groups, such as lysine, arginine, and the like (e.g., histidine). Preferred examples of cationic peptides according to this invention comprise a stretch of at least five, preferably between 5 and 15 positively charged amino acid residues. More preferably, the cationic peptide to be used in the present invention is a peptide of 60 amino acid residues or less, comprising a stretch of at least 5 positively charged amino acid residues. More preferably, the cationic peptide is a peptide of 40 amino acid residues or less, comprising a stretch of between 5 and 15 positively charged amino acid residues. The stretch of positively charged amino acid residues is, in a preferred embodiment, a stretch of one repeated positively charged amino acid residue. Furthermore, in a preferred variant of this invention, at least 50% of the cationic peptide amino acid residues are positively charged, for instance lysine residues.

Cationic peptides of this invention may be for instance polylysine, modified polylysine peptide (for instance chemically modified such as alkylated or gluconoylated), or such as polylysine or modified polylysine flanked by additional amino acid residues. A specific example of such a cationic peptide is a peptide of 13 amino acid residues comprising a stretch of 8 positively charged amino acid residues. Such a cationic peptide is for instance the K8 peptide (Gottschalk *et al*.; 1996), having the sequence YKAK₈ WK (SEQ ID NO: 1). It should be understood that any other cationic peptide having a net positive charge, capable of interacting with a nucleic acid can be used in the present invention.

The fusogenic peptide to be used in the instant invention may comprise 3 to 60 amino acid residues, preferably 5 to 40, more preferably 10 to 30. The term "fusogenic" indicates that the peptide exhibits membrane-disrupting activity, preferably endosomolytic activity, i.e., the ability to disrupt intracellular vesicule compartments and facilitate the release of their constituents in the cytoplasm. Fusogenic peptides generally comprise an amphipathic α-helix with a hydrophobic face (comprising for instance essentially strongly non-polar amino acids) and a hydrophilic face which is dominated by negatively charged amino acid residues (such as glutamic acid for instance). Such peptides may be prepared based on the sequence of known proteins or polypeptides having fusogenic activity, including viral proteins such as the fusion peptide of the influenza virus hemagglutinin, various viral envelope proteins, etc.

A specific example of such a fusogenic peptide is a peptide of 20 amino acid residues comprising an amphipathic α-helix with a hydrophobic face containing only strongly non-polar amino acids and a hydrophilic face which is dominated by negatively charged glutamic acid residues at neutral pH. Such a fusogenic peptide is for instance the JTS-1 peptide (Gottschalk *et al*.; 1996), having the sequence GLFEALLELLESLWELLLEA (SEQ ID NO: 2). It should be understood that any other fusogenic peptide having an endosomolytic activity can be used in the present invention.

In a specific embodiment, the invention comprises the condensation of a plasmid DNA with both the synthetic cationic 13-amino-acid oligopeptide K8 and the fusogenic 20-amino-acid peptide JTS-1. K8 contains a central stretch of eight positively charged L-lysine residues which facilitates the electrostatic interaction of the peptide with DNA. The sequence of JTS-1 was designed as a prototypic pH-dependent endosomolytic peptide and is derived from the fusion peptide of the influenza virus hemagglutinin. A step-wise self-assembly of the DNA with K8 and JTS-1 leads to the formation of electrostatically charged peptide-DNA complexes (also called peptide-oligoplexes). These peptide-oligoplexes are absorbed by cells presumably via receptor-mediated endocytosis, and the endosomolytic activity of JTS-1 promotes the escape of peptide-oligoplexes into the cytoplasm and thus increases the amount of polynucleotide which will reach the cell nucleus. The results presented in the instant invention show that efficient polynucleotide transfer and expression is obtained in ocular cells, both in vitro and in vivo, and that RCS photoreceptor cell degeneration can be significantly delayed by transferring a therapeutic gene to ocular cells via this combined peptide-mediated *in vivo* transfection technique.

In performing the instant invention, the polynucleotide and the peptides are first contacted to form a complex (this step is also termed condensation). In this regard, the peptides can be contacted simultaneously with the polynucleotide, or sequentially. The relative amounts of polynucleotide and peptides can be adapted by the skilled artisan, depending on the contemplated use (in vitro, in vivo, nature of polynucleotide, etc.). It is preferred, however, that the overall charge of the complex be negative to ensure highly efficient polynucleotide delivery and expression in ocular cells in vivo. In this regard, the overall ratio (R) of the positive charges of the peptides on the negative charges of the polynucleotide (+/-)can range from 0.1 to 5, preferably from 0.5 to 1.5, for in vivo uses. More preferably, the invention now shows that efficient in vivo gene delivery in ocular cells is obtained when the above ratio (R) is below about 1, specifically between 0.1 and 1. This aspect is important in performing the instant invention for efficient in vivo use, since prior reports using similar peptide compositions recommended the use of positive overall net charge. For in vitro uses, the ratio R may be higher and reach up to 20, preferably from 0.1 to 15. Furthermore, in preparing the complex, it is also preferred that the molar phosphate (of the polynucleotide) to e-amino groups (of the cationic peptide) be comprised between 0.1 and 5, more preferably between 0.1 and 0.5.

In a particular variant of this invention, the fusogenic peptide and the cationic peptide are coupled together. Coupling can be covalent or non-covalent. Where coupling is covalent, the invention uses a bifunctional peptide comprising a cationic moiety and a fusogenic moiety as defined above. Examples of such bifunctional peptides include any fusion peptide between two peptides as defined above, as well as peptides having both cationic and fusogenic properties such as peptide WEAKLAKALAKALAKHLAKALAKALKACEA (SEQ ID NO: 5) (Wyman et al., Biochemistry 36 (1997) 3008), Gramicidin S (Legendre et al., PNAS 90 (1993) 893), or derivatives thereof, in particular any bifunctional peptide comprising a stretch of positively charged residues and a fusogenic moiety comprising an amphipathic α-helix with a hydrophobic face and a hydrophilic face.

The complex formed between the peptide(s) and the polynucleotide may be used directly for delivery to ocular cells. Alternatively, the complex may be stored in suitable medium and conditions for later usage.

The complex can be contacted with the ocular cells in various ways. For instance, for in vitro or ex vivo polynucleotide delivery, the contacting step generally comprises contacting the cells in culture with the above complex in solution. As an example, 10⁵ cells may be contacted with 1 to 50 µg of complexed polynucleotide, in conventional culture medium (e.g., DMEM, RPMI, etc.) and conditions (e.g., 37°C). The contacting step may last for instance for about 1 to 10 hours. The cells may then be supplemented with fresh medium for further culture. This contacting step may be carried out in any appropriate device (plate, tube, flasc, bottle, etc.), under sterile conditions. For in vivo polynucleotide delivery, the contacting step generally comprises administrering to a subject (mammalian, such as animal or human subject) a composition comprising the above complex (or their constituants). Administration is performed by intra-ocular injection, preferably sub-retinal injection of the composition. Injection can be carried out with any appropriate device, preferably a needle (seringue, etc.), possible upon sclerotomy. The composition used for injection may comprise any suitable pharmaceutically acceptable vehicle, such as sucrose, saline solution, stabilizer, and the like. As an indication, 1 to 40 µg of complexed polynucleotide may be injected per dose. The examples which follow clearly illustrate the efficacy of such compositions for direct polynucleotide transfer and expression into ocular cells in vivo.

The polynucleotide to be used in the instant invention can be any nucleic acid of interest, i.e., exhibiting a biological property. More particularly, the polynucleotide can be any nucleic acid encoding a product (e.g., a polypeptide (including a protein or peptide), RNA, etc.) exhibiting a biological activity. The polynucleotide can be a DNA molecule (cDNA, gDNA, synthetic DNA, artificial DNA, recombinant DNA, etc.) or a RNA molecule (mRNA, tRNA, viral RNA, etc.). The polynucleotide may be single stranded or multiple stranded nucleic acid, preferably double-stranded nucleic acid. The polynucleotide may vary in size from a simple oligonucleotide to larger molecules such as chromosomes for instance, and may be a plasmid, episome, viral genome, phage, an antisense molecule, a gene, etc. In a particular embodiment, the polynucleotide is a double-stranded, circular DNA, such as a plasmid, encoding a product with biological activity. In a more particular embodiment, the polynucleotide is a DNA plasmid encoding a biologically active polypeptide, i.e., a polypeptide which can affect the function, morphology, activity and/or metabolism of ocular cells. More preferred examples of such biologically active polypeptides include trophic factors (in particular neurotrophic factors), anti-angiogenic factors, cytokines, interferons, derivatives thereof and the like. The polynucleotide may also comprise any regulatory signal suitable to provide expression in mammalian cells (e.g., ocular cells), such as promoter region (constitutive, regulated, inducible, tissue-specific, etc.), transcription termination signal, secretion sequences, origin of replication, marker gene, etc., which are available to the skilled artisan. Additionally, the polynucleotide may further comprise nuclear localization signal (nls) sequences, which further enhance polynucleotide transfer to the cell nucleus. Such nls sequences have been described in the literature, including the SV40 large T antigen sequence PKKKRKV (SEQ ID NO: 3) and the bipartite nucleoplasmin motif KRPAATKKAGQAKKK (SEQ ID NO: 4) (Dingwall and Laskey, Trends Biochem. Sci. 16 (1991) 478 ; Kalderon et al., Nature 311 (1984) 33). The polynucleotide can be prepared and produced according to conventional recombinant DNA techniques, such as amplification, culture in prokaryotic or eukaryotic host cells, purification, etc. Furthermore, in a variant of the present invention, a mixture of polynucleotides encoding distinct biologically active products can be used. This variant allows co-expression of different products in the ocular cells.

More preferred polypeptides include trophic factors, in particular neurotrophic factors, such as Fibroblast Growth Factors (acidic, basic, FGF-2), CNTF, BDNF, GDNF, PEDF, NT3, BFGF, derivatives thereof and the like and anti-angiogenic factors, such as ATF, endostatin, angiostatin, the kringle 5 fragment, fragments of fibronectin and fragments of thrombospodin, derivatives thereof and the like. These factors can be of human origin, other mammalian origin or derivatives thereof which retain the desired biological effect. In this respect, various retina-derived neurotrophic factors have the potential to rescue degenerating photoreceptor cells (Li and Turner, 1988a,b; Li *et al*., 1991; Anchan *et al*., 1991; Sheedlo *et al*., 1989, 1993; Guillemot and Cepko, 1992; Steele *et al*., 1993), and may be delivered according to the present invention.

In a particular embodiment of the present invention, the polynucleotide encodes a neurotrophic factor, more preferably a human neurotrophic factor or a derivative thereof, even more preferably a human fibroblast growth factor, or a derivative thereof.

Human basic fibroblast growth factor (hFGF-2; 155 amino acids) is particularly preferred for treating retinopathies such as AMD. In this regard, single intraocular injections of recombinant hFGF-2 were shown to promote the survival of both RPE and photoreceptor cells of RCS rats in a dose-dependent manner for up to 3 months (Faktorovich *et al*., 1990, Perry *et al*., 1995). The addition of hFGF-2, therefore, is sufficient to complement the otherwise reduced concentration of FGF-2 and its receptor in the RCS rat retina (Rakoczy *et al*., 1993; Malecaze *et al*., 1993). Although a direct intraocular injection of hFGF-2 can rescue RCS photoreceptor cells, the effect is transient and the high protein concentration of 0.5-0.8 µg administered lead to cataract formation in all treated eyes (Perry *et al*., 1995). A too high level of hFGF-2 may also favor retinal tumor formation through its stimulating action on cell proliferation (Rogelj *et al*., 1989).

As will be illustrated, the invention can be used in many different areas, in vitro, ex vivo or in vivo. In particular, it can be used to transfer any polynucleotide to ocular cells in vitro or ex vivo. Such transfer may be applied to the production of products (e.g., recombinant proteins), the study of gene regulation or ocular development, etc. In vivo ocular polynucleotide delivery may be used to produce genetically altered animals, to study gene expression in the eye, as well as in therapeutic or prophylactic indications. In this regard, the invention can be applied to the treatment (i.e., the reduction, inhibition or prevention, for instance) of retinopathies (e.g., retinal dystrophies), in particular photoreceptor cell dystrophies, more particularly for retarding photoreceptor cell degeneration.

Among numerous forms of human retinopathies, age-related macular degeneration (AMD) is the leading cause of severe vision loss in the elderly and affects between 6% (New Zealand) and 11% (USA) of people beyond the age of 65 (Kahn *et al*., 1977; Martinez *et al*., 1982; Klein and Klein, 1982). AMD is a heterogeneous group of retinal degenerations and is associated with functional alterations of retinal pigment epithelium (RPE) cells. The congenic Royal College of Surgeons (RCS) rat strain represents the closest rodent model for RPE-linked retinopathies (Bok and Hall, 1971; LaVail *et al*., 1975). Homozygous RCS rats develop a recessive hereditary retinal dystrophy starting in the third postnatal week. This dystrophy is characterized by a progressive degeneration of RPE cells and a secondary loss of photoreceptor cells by apoptosis within the following two months (LaVail, 1981a; Tso *et al*., 1994). The genetic locus for the RCS pathology has been assigned to the centromeric region of chromosome 3 (locus rdy; LaVail, 1981b) which is syntenic to a locus on the human chromosome 20 near the gene encoding the γl-subunit of phospholipase C. The primary degeneration of RPE cells in RCS rats is caused by a deficiency in phagocytosis of outer photoreceptor cell segments which ultimately leads to a massive accumulation of membrane debris in the interphotoreceptor space (Bok and Hall, 1971; Sheedlo *et al*., 1989). Degenerating photoreceptor cells in RCS rats can be rescued transiently by the transplantation of rat RPE cells into the subretinal space (Li and Turner, 1988a,b; Li *et al*., 1991; Gaur *et al*., 1992; Sheedlo *et al*., 1989, 1993). Photoreceptor cells survive also in chimeric rats having both dystrophic and normal RPE cells (Mullen and LaVail, 1976). In these mosaic eyes, patches of neural retina with abnormal and degenerated photoreceptors were present only opposite to patches of non pigmented, mutant pigment epithelium. These findings suggest that RPE cells of RCS rats provide insufficient trophic support to photoreceptor cells.

The instant invention now provides a novel approach allowing an efficient rescue of degenerating photoreceptor cells. In this respect, the results presented in the experimental section show that the instant invention has been successfully applied in vivo to rescue degenerating photoreceptor cells in the Royal College of Surgeons rat. This biological effect was mediated by the subretinal injection of plasmid DNA encoding the human basic fibroblast growth factor (hFGF-2). The DNA condensation with two oligopeptides allowed the negatively charged peptide-oligoplexes to enter efficiently and preferentially choroidal and retinal pigment epithelial cells, which are the preferred target cell populations for AMD treatment. As observed 60 days after transfection, transfected zones revealed a local hFGF-2-mediated delay of photoreceptor cell degeneration. These results clearly show that peptide-mediated ocular cell transfection according to this invention is a novel promising alternative for treating retinopathies (e.g., retinal dystrophies).

Other aspects and advantages of the invention will be disclosed in the following examples, which should be regarded as illustrative and not limiting. All references cited are incorporated in the specification by reference.

### LEGEND TO THE FIGURES

**FIG. 1. *In vitro* transfection of cell cultures using the K8/JTS-1 oligopeptides.** 293 cells were transfected with pREP-EGFP **(A)** or pcDNA-lacZ **(B).** RPE D407 cells were transfected with pREP-EGFP **(C)** or pcDNA-lacZ **(D).**

**FIG. 2. FGF overexpression in transfected RPE-D407 cells.** Western blot analysis of extracts from pREP-5β (**-**) (as negatif control) or pREP-hFGF2 **(FGF)** transfected RPE-D407 cells using an anti-FGF2 antibody. RPE cells were transfected by Fusgene 6 method (Boerhinger). Molecular weights of FGF2 isoforms are indicated (kDa). Asterisks indicate the endogenous FGF2 production (line **-**) which is similar to untransfected RPE D407 (data not shown).

**FIG. 3. Reporter gene expression in transfected normal RCS rdy+p- rat eyes at 3 days post-injection.**
**A-D.** X-gal staining in whole-mounts retinas. **A** : sucrose injected eye (injection volume : 5 µl). **B :** 5 µl of condensed pcDNA-lacZ was injected. Note the local hemorrhages due to the high injection volume (arrows). **C-E.** 1 µl of a pcDNA-lacZ was injected. No local hemorrhages were observed at higher magnification in **D.**
**E :** X-gal staining in a retina cryosection counterstained with red carmine. Choroidal and RPE cell labellings are indicated by arrows and arrow heads, respectively.

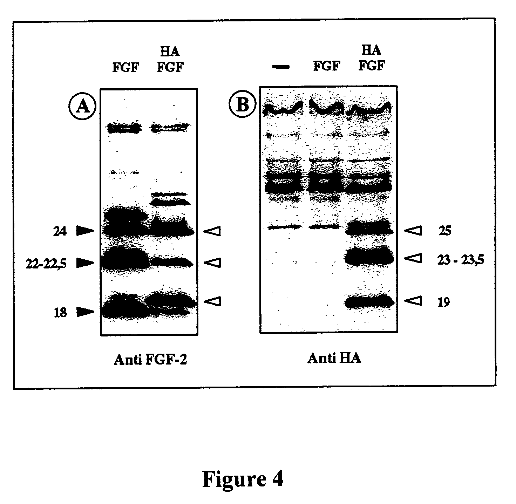

**FIG. 4. HA-hFGF2 fusion proteins expression in transfected RPE-D407 cells.** Western blot analysis of extracts from RPE-D407 cells transfected with pREP-5β (-) (as negatif control), pREP-hFGF2 (**FGF**) or pREP-HA-hFGF2 (**HAFGF**) vectors. RPE cells were transfected by Fusgene 6 method (Boerhinger). **Panel A :** nitrocellulose membrane was treated with anti-FGF2 antibody **(Anti FGF2). Panel B :** nitrocellulose membrane was treated with anti-HA antibody **(Anti HA).** FGF2 isoforms are indicated by filled arrows. Tagged HA-FGF2 isoforms are indicated by unfilled arrows. The molecular weights (kDa) of FGF2 or HA-hFGF2 isoforms are indicated.

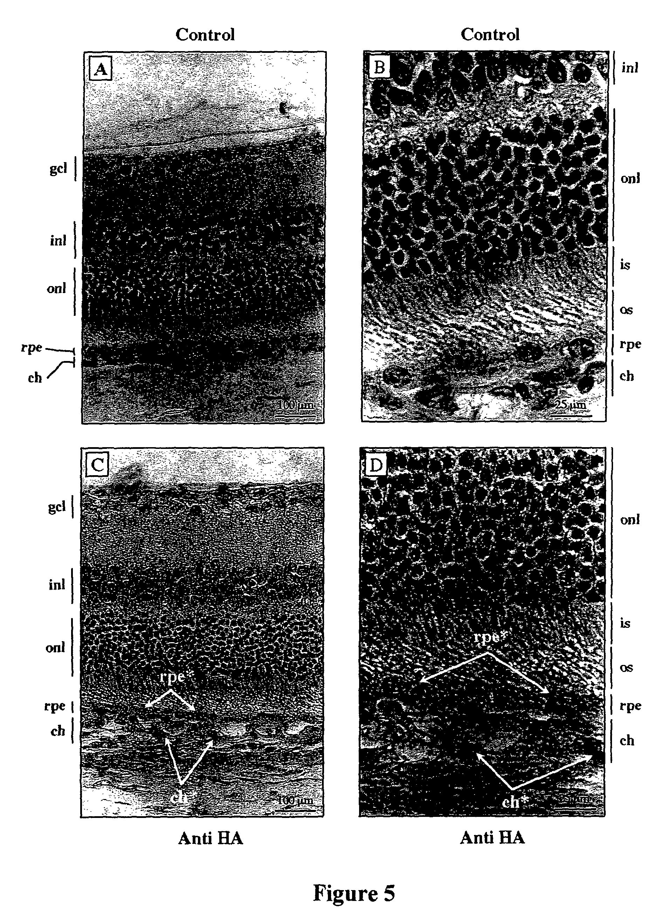

**FIG. 5. Immunodetection of tagged HA-hFGF2 proteins in transfected ocular globes 3 days post-transfection.** Ocular globes of rdy+ p- rats were transfected with pREP-HA-hFGF2 plasmid using the K8/JTS-1 method. **C and D** correspond to embedded paraffin ocular tissue sections treated with anti-HA antibody. The retinal pigment epithelial (**rpe***) and choroidal cells (**ch***) staining is indicated by arrows. **A and B** correspond to the control experiment on adjacent tissue sections treated without primary antibody.
**gcl** = ganglionic cell layer, **inl** = inner nuclear layer, **onl** = outer nuclear layer, **rpe** = pigment epithelial cell layer, **ch** = choroidal cell layer, **is** = inner segment, **os** = outer segment, **rpe*** = HA labelled pigment epithelial cell, **ch*** = HA labelled choroidal cell.

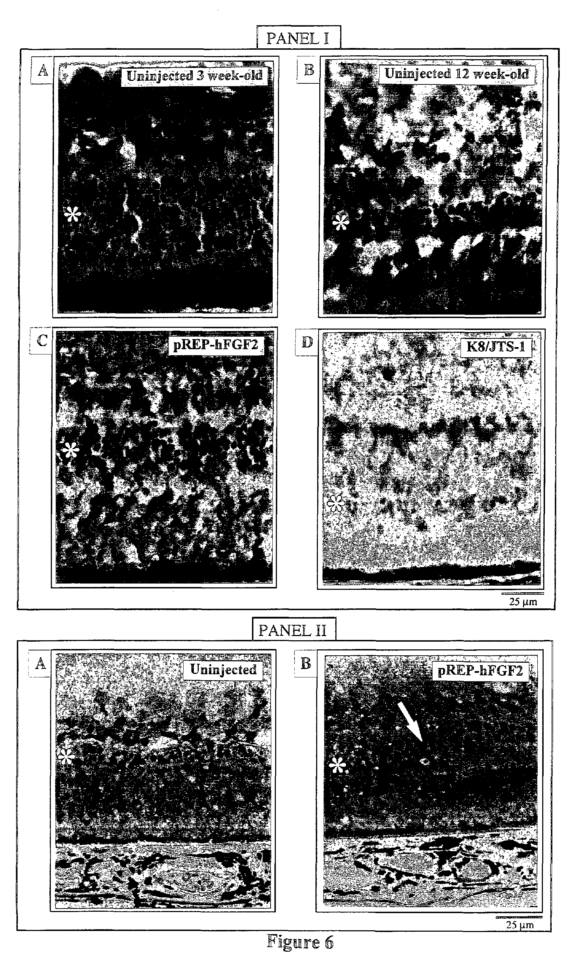

**FIG. 6. Photoreceptor cell rescue by *in vivo* FGF2 cDNA transfection.**
**Panel I-II :** Toluidine blue stained retina from untreated and treated dystrophic RCS rat, sixty days after injection. (cryosections 16 µm).
**I** - Cryosections from untreated, pREP-hFGF2-transfected or vehicle-injected dystrophic RCS rat eyes (group II, see materials & methods). **A-B.** 3-week-old **(A)** and 12-week-old **(B)** untreated RCS rdy- p+ rat retina. It should be noted that 12-week-old RCS rdy+p+ rat retina present the same aspect than 3-week-old RCS rdy- p+ rat retina (data not shown). **C-D.** hFGF2-transfected **(C)** and vehicle-injected **(D)** retina of a 12-week-old RCS rdy- p+ rat. Asterisks indicate the photoreceptor cell layer. Vehicle: K8/JTS-1 in 0.25 M sucrose. Note the 5 photoreceptor cell rows in pREP-hFGF2 transfected eyes compared to the 2 photoreceptor cell rows in vehicule injected eyes
**II -** Epon sections of 2 µm from untreated or pREP-hFGF2-transfected dystrophic RCS rat eyes. **A.** Retina of untreated RCS rdy-p+ eyes. **B.** Retina section very close to the injection sites of eyes transfected with pREP-hFGF2. Locally restricted areas of photoreceptor cell rescue is indicated by arrow.

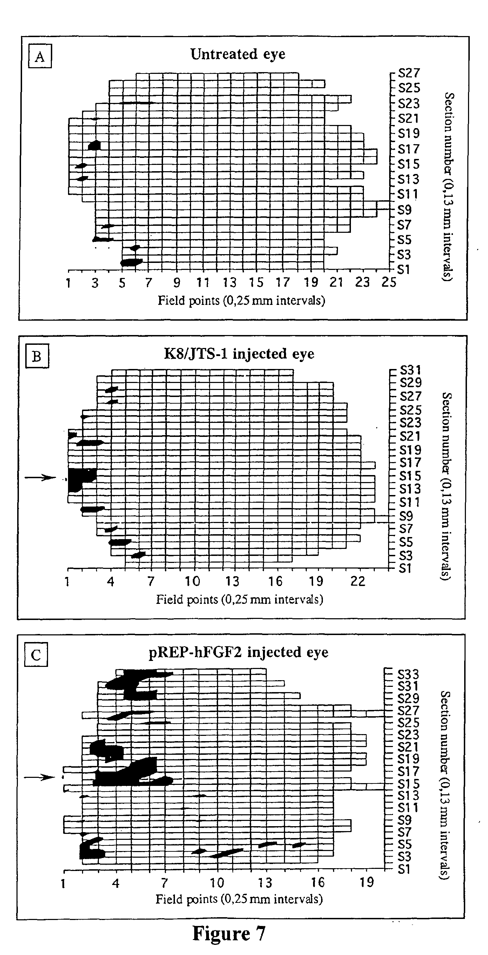

**FIG. 7. Schematic representation of the dystrophic retina from 12-week-old RCS rdy-p+ rats.** Equidistant cryosections (one section every 130 µm) were performed on the entire retina surface. Graph vertical axes correspond to section numbers. Graph horizontal axes represent the analysed observation fields on each section (one analyse every 250 µm). Black areas represent the retinal surface which fulfill the criteria for delayed cell degeneration (see materials and methods, histological analysis). Typical retinal areas with delayed photoreceptor cell degeneration in an untreated **(A),** vehicle-injected **(B),** and pREP-hFGF2-transfected eyes **(C).** Vehicle: K8/JTS-1 in 0.25 M sucrose. Injection volume: 1 µl. Injection sites are indicated by arrows.

### MATERIALS AND METHODS

### Plasmids construction :

The pREP-hFGF2 encodes four FGF2 isoforms (18, 22, 22.5 and 24 kDa) produced by alternative translation. Human FGF2 (hFGF2) open reading frame (ORF) was excised from the pSCT40 plasmid (kindly provided by Dr. H. Prats) with the restriction enzymes *Xho*I and *Hind*III and inserted downstream to the Rous Sarcoma Virus (RSV) promoter into the *Xho*I/*Hind*III restriction sites of pREP5β (Lei *et al*., 1996) (kindly provided by Dr. D.C. Gruenert). The pREP-HA-hFGF2 plasmid encodes four FGF2 isoforms in fusion with HA epitope (HA is a 11 amino acid peptide of influenza hemagglutinin). A PCR amplification was done on the pREP-hFGF2 using HA-FGF 5' sense and FGF 3' antisense oligonucleotides. The NcoI-BamHI restriction PCR fragment was inserted in pREP-FGF vector into the NcoI-BamHI sites instead of wild type hFGF2 sequence. The HA sequence is localized just below the ATG initiation codon of the 18 kDa FGF2 isoform : HA-FGF 5' sense oligonucleotide : 5'AAAA**CCATGG**CTTACCCATACGATGTTCCAGATTACGCTAGCGCAGCCGGGAG CATCACC3' (SEQ ID NO: 6) ; FGF-3' antisense oligonucleotide : 5'GACCAATTATCCAAACTGAGCTT3' (SEQ ID NO:7), Nco I site is in bold, HA epitope sequence is underlined. The pREP-EGFP plasmid encodes the genetically modified 23 kDa form of the green fluorescent protein (GFP) from the jellyfish *Aequorea victoria* (Zolotukhin *et al*., 1996). The *Bam*HI*-Not*I restriction fragment coming from pEGFP-N1 (Clontech Laboratories, Palo Alto, USA) was inserted into the *Bam*HI*/Not*I sites of pREP5β. In pcDNA-lacZ, the LacZ transgene expression is driven by the cytomegalovirus enhancer/promoter sequence (kindly provided by Dr. D. Blondel).

### Cell culture:

All culture plates and dishes were purchased from Techno Plastic Products (Däniken, Switzerland) whereas culture media and their supplements were provided by Life Technologies (Paisley, UK). Growth medium is Dulbecco's Modified Essential Medium DMEM/F12 containing 100 units/ml penicillin, 0.1 mg/ml streptomycin, 0.25 µg/ml Amphotericin B and enriched by 10% decomplemented Australian fetal calf serum (FCS).

### In vitro cell transfection

### DNA condensation :

The two synthetic oligopeptides K8 and JTS-1 were purchased from Dr. J.T. Sparrow (The Methodist Hospital, Houston, Texas, USA). 2.4 µl of an aqueous solution of K8 (8.0 mg/ml) was added to 10 µg plasmid DNA dissolved in 2 ml 0.25 M sucrose, and incubated in Sarstedt tubes for 30 min at room temperature (molar phosphate to ε-amino group ratio: 1/30). Then, 4.6 µl JTS- 1 (dissolved in 0.25 M sucrose and adjusted to pH 7.5 with 11.5 mM HEPES pH 7.5 plus 1 N NaOH) were added to obtain an overall +/- charge ratio of 10,2 (Gottschalk *et al*., 1996) and incubated for another 30 min at room temperature. Cell transfection was carried out within 60 minutes after the end of the condensation procedure.

### DNA transfection :

Adherent cultures of 293 cells or human RPE D407 cells were prepared in 6-well culture plates (10⁵ cells/well). Cell cultures were transfected with 10 µg of each peptide-condensed plasmids during a 4 h incubation with peptide-oligoplexes in 2 ml DMEM/F12 at 37°C in a wet 5% CO₂ incubator. Then, 2 ml of DMEM/F12 plus 10% FCS were added for 72 h.

### Determination of the transfection efficiency :

293 and human RPE D407 cells were transfected with peptide-condensed pREP-EGFP (10 µg) or pcDNA-lacZ (10 µg). The percentage of X-gal stained cells or fluorescent cells were estimated as relative to the total number of cells. Cytotoxicity and influence on the proliferation rate were evaluated by comparing the total cell number of transfected versus untreated cell cultures.

### Western blots analysis

RPE D407 cells were transfected with FuGENE 6 according to the manufacturer's instruction *(Boehringer, Germany)* with pREP5β, pREP-hFGF2 and pREP-HA-hFGF2 vectors. 48 hours after transfection, the transfected cells were selected for five days with hygromycin (200 µl/ml ; *Boehringer, Germany).* Then, they were lysed in RIPA buffer (150 mM NaCl, 1 % NP-40, 0,5 % DOC, 2% SDS, 50 mM TRIS, pH 8). Total proteins were quantified by Bio-Rad protein assay. 40 µg of proteins from each cell lysate were separated in 15 % polyacrylamide gel (pH 8,8) and transferred onto a nitrocellulose membrane. This membrane was treated with a rabbit polyclonal anti-FGF2 antibody (Oncogene science,USA) or a polyclonal rabbit HA-11 antibody (Babco, USA). Antibodies were detected using an enhanced chemiluminescence (ECL) kit according to the manufacturer's instruction (Amersham Pharmacia Biotech, UK).

### ELISA Protein Assay from ocular globes:

Cornea and lens of each rat eyes transfected with pepide-condensed pREP-5β, pREP-hFGF2 or untransfected were removed and eyes were homogenized mechanically in 200 µl hypotonic lysis buffer (5 mM sodium phosphate buffer at pH 7.0, 30 mM NaCl, 0.1% Tween-20, 1 mM phenylmethylsulfonyl fluoride, 1 µM leupeptin, 1 µM pepstatin). Following centrifugation at 15,000 g for 10 min, the amounts of total proteins and FGF2 were determined in the supernatant by Bradford Protein Assay (Bio-Rad Laboratories, München, Germany) and ELISA (Quantikine; R&D Systems, Abingdon, UK; Dynatech MR5000 reader with 450 nm test filter and 550 nm reference filter), respectively.

### Animals

All animals were handled in strict accordance with the Helsinsky Declaration and with the Association for Research in Vision Ophthalmology Statement for Use of Animals in Ophthalmic and Vision Research. The animals were kept in environment at 21°C with a 12h light (100 lx): 12h dark cycle and fed *ad libitum.* Normal pigmented (p+) or non-pigmented (p-) RCS rats which showed no retinal dystrophy (RCS rdy+) as well as dystrophic (RCS rdy-) rats were kindly provided by Dr. M.M. LaVail (UCSF, San Francisco, USA). Postoperatively, all rats were kept under the same housing conditions.

### In vivo cell transfection

### DNA condensation :

The *in vivo* protocol was different from the *in vitro* protocol only in the 1,4 µg of DNA which was peptide-condensed in 2 µl of 0,25 M sucrose. All transfections were carried out with peptide-condensed plasmids (1.4 µg DNA/µl 0.25 M sucrose) with an overall +/- charge ratio of 0.73 and a phosphate to ε-amino group ratio of 1/3.

### Trans-scleral subretinal injections :

Adult rats were anesthetized by an intraperitoneal injection of 0.25-0.5 ml Nesdonal (Rhône-Poulenc Rorer, Vitry-sur-Seine, France) whereas 3-week-old rats were anesthetized with 100 mg/kg ketamine (Imalgène 1000; Rhône Mérieux, Lyon, France). The cornea was topically anesthetized by a drop of oxybuprocaine chlorhydrate (Novésine 0.4%; Merck Sharp & Dohme-Chibret Laboratoires, Paris, France) to inhibit the eyelid reflex. The temporal superior region of the conjunctival tissue was cut 1 mm behind the limbus. Preliminary sclerotomy was performed at this site by means of a surgical monofilament needle (10/0). Then, a loaded 10 µl Hamilton syringe with a blunt 33-gauge needle was introduced tangentially into the punctured eye. Injections were performed manually at the subretinal level under randomized conditions by the same experimentator. Treated and untreated eyes were covered with Sterdex paste (Ciba Vision, Basel, Switzerland) containing dexamethasone and oxytetracycline.

### HA immunohistochemical staining :

pREP-HA-hFGF2 and pREP-hFGF2 transfected globes of RCS rdy+p- rats were fixed in Davidson (Prolabo®) for 24 hours after the sacrifice of the injected rats. The globes were dehydrated in ethanol, embedded in paraffin, sectioned at 5 µm and mounted on superfrost + slides (Fisher). Sections were incubated with 3% H₂0₂ during 10 minutes, washed three times in PBS (phosphate Buffer Saline) and then incubated for 15 minutes with normal swine serum (DAKO, France). They were subsequently incubated with primary anti-HA antibody (BabCo, USA) at a dilution of 1/1000 in PBS, 0.3% Triton, 5% normal swine serum, overnight, at +4°C. After four washings in PBS, slides were incubated with HRP-coupled secondary antibody (DAKO, France) for 30 minutes at room temperature. HA staining was visualized using a Peroxydase/Diaminobenzidine detection Kit (DAKO, France). Sections were finally counterstained according to the Harris hematoxylline coloration method and mounted with Eukitt (Polylabo, France).

### Tissues preparation for transfection efficiency test:

### - Animal group I - Reporter gene expression:

Transfection with reporter genes was performed in adult RCS rdy+p- rats which received a single subretinal injection of 1-5 µl of either 0.25 M sucrose, uncomplexed, or K8/JTS-1-condensed plasmids (pcDNA-lacZ, pREP-EGFP). Injected rats were sacrificed by CO₂ asphyxia and their eyes were enucleated *post-mortem*. The lens and the vitreous body were removed before fixation.

***For EGFP detection :*** the eyes were fixed in 2% paraformaldehyde/PBS, 1 hour at room temperature. After washing in PBS, specimens were flat-mounted in PBS. The cellular distribution of the enhanced green fluorescent protein (EGFP) was visualized by fluorescence microscopy at an excitation wavelength of 490 nm.

***For β-galactosidase coloration :*** the eyes were fixed in 0.5% glutaraldehyde/PBS plus 0.001% Triton X-100, 2 hours at room temperature. After washing in PBS, β-galactosidase expression was analyzed according to two different techniques:
- Retina were flat-mounted in PBS and X-gal reaction was performed as described by Ribeaudeau *et al*. (1997).
- Globes were cryoprotected in 15% sucrose/PBS for 24 h at 4°C and embedded in Tissue-Tek O.C.T. compound. Serial 16-µm-thick cryosections were performed, X-gal reaction was realized as described by Ribeaudeau *et al*. (1997), and cryosections were stained with 1% (w/v) carmine (Natural Red 4; Sigma-Aldrich Chemie GmbH, Steinheim, Germany) in 4% (w/v) potassium aluminum sulfate for 1 min. Then, the sections were washed in distilled water and coverslipped in Aquatex (Merck, Darmstadt, Germany).

### Tissues preparation for photoreceptor survival and toxicity studies in RCS rat:

### - Cryosection analysis - Animal group II:

Twelve 3-week-old RCS rdy- p+ rats were transfected with 1 µl of either K8/JTS-1 in 0.25 M sucrose (vehicle; n=6) or K8/JTS-1-condensed pREP-hFGF2 (n=6). 60 days post-transfection, rats were sacrified, globes cryosections were stained with 0.1% toluidine blue in 0.2% sodium borate for 90 sec., washed in 5% ammonium heptamolybdate for 5 min., and dehydrated in ethanol before they were coverslipped in Eukitt (O. Kindler GmbH, Freiburg, Germany).

### - Epon analysis - Animal group III:

Three groups with 3-week-old RCS rdy- p+ rats either remained untreated (n=4) or injected with 1 µl of the condensed pREP5β (n=5) or pREP-hFGF2 (n=6) plasmids. Sixty days post-injection, rat organs were fixed by transcardial perfusion with 2.5% glutaraldehyde (Merck, Darmstadt, Germany) in 0.2 M sodium phosphate buffer pH 7.3. Their eyes were post-fixed in 2% osmium tetroxide (Electron Microscopy Science, Fort Washington, PA, USA) and embedded in epon 812 according to Luft (1961). 2 µm sections were then counterstained with toluidine blue.

### - Toxicity study - Animal group IV :

3-week-old RCS rdy+p+ rats were transfected with 1 µl of either condensed pREP5β (n=5) or pREP-hFGF2 (n=6) plasmids. Sixty days post-injection, rats were fixed like the group III but the eyes were post-fixed overnight in Davidson's fixative and embedded in paraffin. 5 µm sections were then counterstained with toluidine blue.

### Histological analysis :

In rats of group II and III, the number of photoreceptor cell layers were counted at injection site, with a light microscope at a 400-fold magnification on sections about 130 µm apart. In each section, the number of photoreceptor cell rows in the outer nuclear layer was determined at equidistant field points being separated by 250 µm intervals across the retina. If a field point revealed a row number with more than double of the minimal number of photoreceptor cell layers, observed in the same section, it fulfilled the rescue criterion for delayed photoreceptor cell degeneration. The field of delayed cell degeneration was determined on two-dimensional schematic representations of the retina (Fig 7). It corresponds to the area outlined by field points which fulfilled the rescue criterion. In addition to the number of photoreceptor cell layers on epon sections (group III), the integrity of retina and Bruch's membrane, retinal pigment cell translocation, subretinal neovascularization, and subretinal macrophage infiltration were examined at the injection sites and elsewhere throughout all the examined tissue sections. Statistical analyses were performed on the amount of rescued field points (group III) or on their percentage data (group II). All data passed the Kolmogorov-Smirnov test for normal distribution. In both series, one-way ANOVA gives significant differences between groups of treated animals (P<0.01). A two-way ANOVA of the series II revealed that inter-individual differences between rats had no influence on the photoreceptor cell rescue effect of the treatments (P=0.17). These analyses allow the application of two-tailed unpaired Student's t-tests to compare the effect of the different treatments on photoreceptor cell rescue. Welch's correction of the data was performed in cases of significantly different variances. Sections of the experimental group IV were analyzed qualitatively at the injection sites and throughout all the examined retinal tissue sections with respect to the occurrence of retinal or choroidal atrophy, neovascularization, rupture of Bruch's membrane, and subretinal or intraretinal macrophage infiltration.

### RESULTS

### In vitro K8/JTS-1 mediated transfection in RPE-D407 and 293 cells :

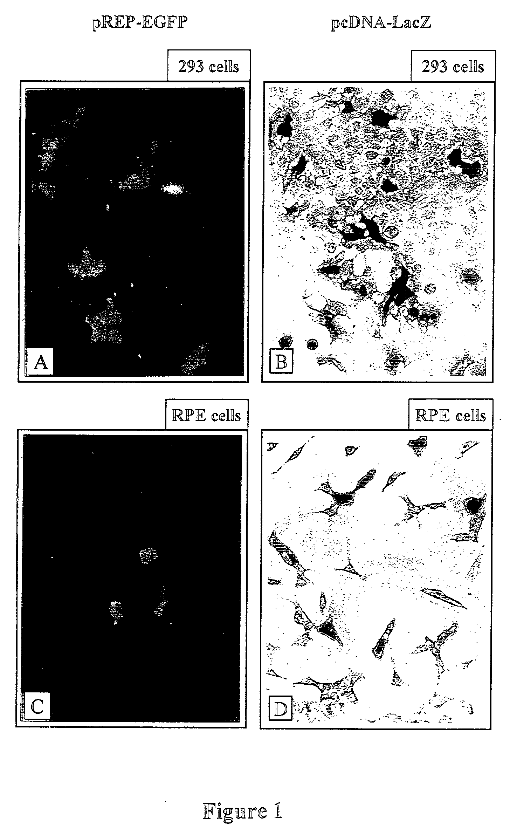

In order to test the k8/JTS-1 *in vitro* transfection efficiency, the episomal plasmids pREP5β and pREP-EGFP as well as the plasmid pcDNA-lacZ were transferred to 293 and RPE D407 cells (Fig. 1). No apparent transfection-mediated cytotoxicity was observed in 293 cells. The peptide-mediated DNA transfer resulted in similar transfection efficiency of 18.5 ± 3.5% (S.E.M.; n=4) for 293 cells and 11.1 ± 1.3% (S.E.M.; n=4) for RPE D407 cells whatever the reporter gene used. These results confirm the *in vitro* efficiency of the K8/JTS-1 derived method for transfection of RPE cells.

### In vitro expression of the human FGF2 transgene :

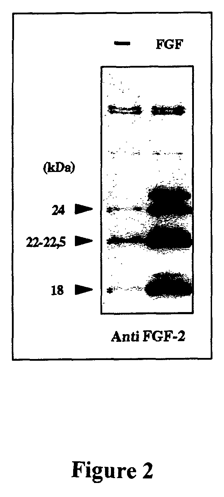

The pREP-hFGF2 plasmid was constructed by introducing the FGF2 cDNA in the pREP5β plasmid. The expression of the hFGF2 transgene was verified by western blotting in RPE D407 cells. As expected, four FGF2 isoforms are efficiently overexpressed (see Fig 2 ; line FGF). It should be noted that basal endogenous FGF2 expression is observed (Fig 2 ; line-). Thus, *in vitro,* the pREP-hFGF2 plasmid allow an efficient overexpression of all FGF2 isoforms and in particular the secreted hFGF2 isoform of 18 kDa.

### In vivo transfection of the reporter genes LacZ and EGFP :

### Determination of the optimal ratio of K8/JTS-1 peptide for in vivo ocular transfection :

As a first step, we determined the apropriate K8/JTS-1 ratio to obtain an efficient *in vivo* transfection. To this purpose, seven ocular globes of RCS rdy+p- rats received subretinal injections of 5 µl pREP-EGFP plasmid which was either uncomplexed or peptide-condensed with a molar phosphate to ε-amino group ratio of 1:3 and an overall +/- charge ratio of 0.73, 0.75, 0.80, 1.19, 1.29, or 1.33. Efficient EGFP transgene expression was detected only in globes transfected with negatively charged peptide-oligoplexes (data not shown). Among them, the peptide-pREP-EGFP complex with the highest negative net charges (overall +/- ratio: 0.73) revealed the best transfection efficiency and, therefore, was used in the subsequent expression analysis.

### In vivo reporter transgene expression (animal group I):

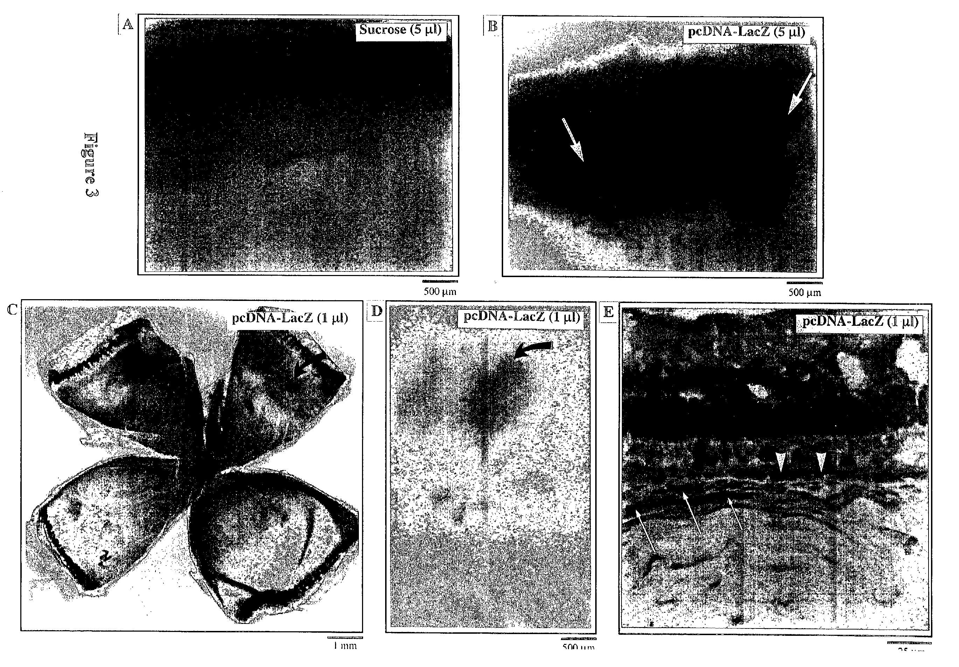

Subretinal injections were performed in ocular globes of RCS rdy+p- rats with 7 µg of condensed pcDNA-lacZ diluted in a volume of 1 or 5 µl. As a control, eyes were injected with sucrose only. The reporter gene expression was monitored 3 days post-injection by β-galactosidase-mediated X-gal coloration (Fig. 3). As shown in Figure 3-A, X-gal staining was absent from sucrose-injected eyes. However, the injection of 5 µl of pCDNA-LacZ revealed a wide zone of lacZ transgene expression around the injection site (Fig 3B) visible through the pink sclera as well as after removal of the neural retina. Area of the transfected region is typically of 1 mm² which corresponds to approximately 2% of the ocular surface. To minimize surgically induced trauma, we injected 1 µl of the condensed-pcDNA-lacZ corresponding to only 1,4 µg of DNA. This low DNA quantity was used in order to avoid precipitation of the DNA-peptide complexes. The area of cell transfection was strongly reduced as compared with 5 µl injections and the β-galactosidase-producing cells are confined to a small region at the injection site (Fig. 3C, arrows). Meanwhile, retinal detachment and local hemorrhages were strongly reduced (Fig 3D). On a 16-µm-thick cryosections through the same transfection site, cytoplasmic X-gal staining is visible in choroidal and RPE cells (Fig. 3E). In other ocular layers such as the retina or sclera, no transgene expression was observed. The same results was obtained using the pREP-EGFP vector (data not shown).

### In vivo expression of the FGF2 transgene :

### Quantification of the FGF2 transgene expression :

21-day-old RCS p+ rats were injected with 1 µl corresponding to 1,4 µg of condensed pREP-hFGF2. Condensed pREP5β or the K8/JTS-1 oligoplexes alone were injected as controls. Two days after injection, the amount of FGF2 in these injected globes as well as untreated eyes was analysed by ELISA and expressed as relative to the amount of total proteins. Untreated eyes produced between 0.26 ng and 0.34 ng of rat FGF2 which corresponds to an average of 5.1±0.4% (S.E.M.) of total proteins. As compared to these eyes, ocular globes that received injections showed a significantly higher FGF2 production of 0.24-0.88 ng or an average amount of 9.1 ±0.8% (S.E.M.) of total proteins (P=0.0005; two-tailed unpaired Student's t-test) whatever the solution injected (P=0.759; one-way ANOVA).

### Localization of the FGF2 transgene expression :

In order to visualize the production of hFGF2 in transfected ocular cells, we used the plasmid pREP-HA-hFGF2. This plasmid encodes the four FGF2 isoforms tagged with the HA peptide. As shown by western immunoblotting using anti-HA or anti-FGF2 antibodies, human RPE D407 cells transfected with pREP-HA-hFGF2 produce the full-length HA-FGF2 fusion proteins (Fig 4, line HAFGF). This vector was thus transfected *in vivo* in four rdy+p- rats as described above. As expected, the subretinal injection of peptide-condensed pREP-HA-hFGF2 leads to the local production of the tagged HA-hFGF2 mainly in choroidal and

RPE cells (Fig. 5 C-D). These results confirm the effective expression of the FGF2 transgene in RPE cells after K8/JTS-1 mediated transfection.

### Photoreceptor cell rescue by in vivo FGF2 cDNA transfection

In dystrophic RCS p+ rat eyes, the photoreceptor cell degeneration starts around the third postnatal week by a gradual reduction of the initial 10 photoreceptor cell layers (Fig. 6A). Typically, only 2-3 photoreceptor cell layers are conserved in an 12 week old RCS rats (Fig. 6B). However, the loss of photoreceptor cells is slower in peripheral areas proximal to the ora serrata.

### Cryosection Study:

As the FGF2 was previously shown to be able to partially rescue the photoreceptor cell degeneration, we decided to test the impact of the K8/JTS-1 mediated hFGF2 transfection on this process. Two groups of 21-day-old RCS rdy- p+ rats received single subretinal injection of peptide-condensed pREP-hFGF2 (n=6) or K8/JTS-1 (vehicle; n=6) in 0.25 M sucrose (see material and methods, group II). Sixty days after injection, the retina of treated and untreated eyes were frozen. Retinas were cut in 16 µm sections every 130 µm and the number of photoreceptor layers was counted on each sections. Rescue area was defined as region containing at least more than twice the minimum number of photoreceptor cell layers observed in the same section. We observed a significant increase of the rescue area in the hFGF2 transfected eye (14.5% of all examined field points) compared to their untreated contrelateral eye (2,65 % ; P=0,001, Student's t-test) or the vehicle injected eyes (3,98 % of all examined field points ; P=0.002, Student's t-test) (see Table 1 ; Fig. 6, panel I and Fig. 7). This area corresponds approximately to 1 mm² which is equivalent to 2 % of the entire retina surface and is 10-14 times larger than that seen in vehicule injected globes (Fig 7). In zones corresponding to the injection site, a small increase in photoreceptor cell rescue is observed, even in the vehicule injected eyes. However, the amount of rescued filled points in vehicules-injected eyes (3,98 %) doesn't differ significantly from that observed in contralateral retina of the eye injected with the vehicle (4,05 % ; P=0.961, Student's t-test) or the pREP-hFGF2 plasmid (2,65 % ; P=0.149, Student's t-test) (Table 1).

### Epon study:

To confirm cell rescue due to the production of hFGF2 mediated by the transfection *via* pREP-hFGF2, rescue effect of pREP5β and pREP-hFGF2 was further examined in epon-embedded RCS rdy- p+ rat eyes (see materials and methods, group III). 60 days post-transfection, sections of pREP-hFGF2-transfected globes revealed zones of rescued photoreceptor cells at the transfection site which covers 17.5 % ± 2.2 (S.E.M.; n=6) of the retinal surface (Table 1 ; Fig. 6 , panel II-B). As compared to this experimental group, a significantly smaller zone of photoreceptor cell rescue was found in pREP5β-transfected (2.6 % ± 1.2 (S.E.M.), n=5; P=0.0025, Student's t-test) and non-injected control eyes (1.3 % ± 0.5 (S.E.M.), n=4; P=0.0026, Student's t-test) (Table 1 ; Fig 6, panel II-A).

We thus demonstrate that the K8/JTS-1 mediated hFGF2 transgene expression in RPE and choroid cells induce a significant delay in photoreceptor cell degeneration.

### In vivo side effect of the K8/JTS-1 mediated transfection :

The effect of FGF2 cell transfection on the integrity of both retinal cell layers and Bruch's membrane as well as on subretinal neovascularization and macrophage infiltration was further examined at the injection sites in epon-embedded RCS p+ rat eyes (see materials and methods, group III). At the injection site, a rupture of Bruch's membrane was generally observed and occasionally a very local photoreceptor cell atrophy. Likewise, signs of wound healing such as subretinal or choroidal fibrosis were also focally detected in 1 of 6 rats which received the pREP-hFGF2 plasmid, in 4 of 5 pREP5β transfected rats. At proximal surroundings of the injection zone, injection-mediated cytotoxicity, apparent as local choroidal atrophy or retinal necrosis, was observed in 2 of 6 pREP-hFGF2 transfected rats, in 4 of 5 rats of the pREP5β treated rats, and in 4 of the 5 pREP-EGFP treated eyes. Enhanced subretinal neovascularization, appearing typically as a local accumulation of capillaries, occurred also strictly around the injection site whatever the construct used. As compared to non-injected control eyes with an average of 0,8 ± 0,5 (S.E.M., n=4) neovascularization sites, significantly more such sites were found in the injection zones of pREP5β transfected rat eyes (9,8 ±1.7 (S.E.M), n=5; P=0.008, student's t test) and pREP-hFGF2 transfected eyes (8.0 ± 2.0 (S.E.M.), n=6; P=0.018, Student's test). A translocation of retinal pigmented cells, normally attached to Bruch's membrane, towards the inner retinal layers was observed not only at the injection sites but irregularly distributed over the whole retina in all rats examined (data not shown). On the other hand, in the two experimental groups of rats which received injections, no obvious accumulation of macrophages or microglial cells were observed at the injection sites neither at distance from the injection sites.

### FGF2 in vivo transfection doesn't induce cytotoxic effect :

To examine a possible toxic side effect of transfection on retinal cells, RCS rdy+p+ rats eyes were transfected with condensed pREP5β (n=5) or pREP-hFGF2 (n=6) eyes (see materials and methods, group IV). As determined at 60 days post-injection, on paraffin embedded ocular globes, the injection of pREP5β resulted in a very local photoreceptor and RPE cell atrophy in 3 of 5 globes. The injection of pREP-hFGF2 caused a similar type of local retinal atrophy in 2 of 6 eyes. Among these eyes, one globe revealed a rosette-like rearrangement of photoreceptor cells at the injection site.

Meanwhile, in all eyes examined, we couldn't find any intraocular microglia infiltration, cataracts, or any surgically induced eye infection. Furthermore, tumors or organ malformations were absent from other body parts of all treated rats, as determined by macroscopic and microscopic examinations. So, although minor side effects were focally detected at the injection site due to chirurgical trauma, we didn't observed any major toxic side effects with this *in vivo* peptide-mediated ocular cell transfection.

### DISCUSSION

### K8/JTS-1-mediated cell transfection

In the present study, we describe an *in vivo* transfection protocol which is efficient for the transfer of plasmid DNA into ocular rat tissues. This non-viral technique is based on the condensation of circular DNA with the two peptides K8 and JTS-1 and is particularly suitable for the transfer of large molecules such as the episomal plasmid pREP5β (Gottschalk et al., 1996). Using the temporal trans-scleral injection approach, we were able to transfect ocular cells with peptide-condensed transgenes encoding either the two reporter gene products β-gal and EGFP or the human trophic factor (hFGF2).

As shown by the two reporter genes expression, an injection of peptide-condensed plasmid DNA into the subretinal space leads to the transfection of mainly choroidal and RPE cells whereas other retinal cells appear to be untransduced. Choroidal cells are normally separated from the retina by Bruch's membrane. Their transfection is likely due to the local perforation (seen on epon-embedded sections) caused by the sub-retinal injection. It seems that the surgical injection procedure but not the peptide-oligoplexes, may display a certain degree of cytotoxicity *in vivo.* Side effects, independant from the plasmid injected, such as choroidal atrophy and enhanced subretinal neovascularization were observed in proximal surroundings of injection sites. The area of cell transfection typically covers 0,72 mm² which corresponds to 2-3% of the total retinal surface.

### Effect of in vivo transfection on photoreceptor cell degeneration

The therapeutic capacity of the K8/JTS-1 transfection system to rescue degenerating photoreceptor cells was determined in the RCS rat model which is widely used to study RPE-induced retinal dystrophies. We have shown that the degenerating retina can be rescued by a local transfection of ocular cells with the hFGF2-encoding plasmid pREP-hFGF2 complexed with JTS-1/K8 peptides.

*In vivo*, the observed rescue effect confirms previous studies with recombinant hFGF2 showing that this protein can delay photoreceptor cell degeneration (Faktorovich et al., 1990, 1992; Akimoto et al., 1999; Uteza et al., 1999). Our findings indicate that, due to the 97% sequence similarity to its rat homolog, hFGF2 retains its property to effectively act on RCS retinal cells. Its action is likely to be mediated by the high-affinity binding of hFGF2 to the rat transmembrane tyrosine kinase receptors and may trigger a variety of cellular rescue mechanisms such as a local restoration of phagocytosis in transfected RPE cells (McLaren and Inana, 1997), cell repair (Fernig and Gallagher, 1994), and inhibition of both apoptosis (Tso *et al*., 1994; Guillonneau et al., 1997; Karsan *et al*., 1997) and the production of cytotoxic nitric oxide in RPE cells (Goureau *et al*., 1993a,b). It remains to be determined by multifocal electroretinogram recordings whether the surviving photoreceptor cells maintain their ability to transduce light signals and transmit them to higher synaptic integration levels of the visual system.

Although others have reported that ocular injury itself can inhibit RCS photoreceptor cell degeneration (Faktorovich et al., 1990; Wen et al., 1995), due to induction of the endogenous neurotrophic factor expression, we found very little difference in the area of delayed photoreceptor cell degeneration between vehicle-injected and untreated control eyes. It may be possible that our analysis method on cryosections was not sensitive enough to detect a rescue effect produced by the injection procedure itself. We, therefore, studied the rescue effect on epon embedded sections after 60 days post-transfection. In these tissue preparations, we found again the most pronounced local rescue effect on photoreceptor cells when the ocular cells were transfected with the hFGF2 transgene. In globes injected with vehicle or the pREP5β control plasmid, the rescue effect was either absent or confined to a smaller retinal zone. The endogenous production of homologous rat FGF2 enabled us to quantify *in vivo* by ELISA the low level local transgene expression. In order to verify that the observed rescue was specifically due to the transgenic hFGF2 expression, we used a plasmid encoding an HA tagged FGF2 protein. After injection of this plasmid, the expression of the recombinant protein was observed in choroidal and RPE cells. We thus assumed that the rescue is correlated to an increase of the FGF2 transgene expression. So, the recombinant hFGF2 protein presumably secreted by these cells may counteract the otherwise lowered endogenous concentration of FGF2 in the RCS photoreceptor cell layer (Rakoczy *et al*., 1993).

The rescued area induced by hFGF2 is larger than the area of transfection observed in eyes transfected with similar volumes of reporter genes. This observation suggests that the trophic action of this factor may have diffused from transfected choroidal and retinal pigment epithelial cells towards neighboring retinal zones. For instance, the trophic effect of hFGF2 on photoreceptor cells may have been amplified and/or relayed to a broader range of retinal cells via stimulation of FGF2 production in Müller cells, as it has been shown in culture by Cao et al. (1997). In addition, the absence of intraocular tumors or cataracts from hFGF2-transfected globes suggests that the low level of production of this trophic factor by transfected cells did not induce tumor-like ocular cell proliferation.

In other *in vivo* previous reports, recombinant FGF2 expression was controlled by the RSV promoter which is active in adenovirus-infected RPE cell culture for at least 3 weeks (Li and Davidson, 1995) or in transplanted fetal brain cells for 1-2 months (Jiao *et al*., 1992). Similarly, we found that RSV promoter-driven reporter gene expression in ocular cells is detectable 6 weeks after transfection. However, the level of expression decreased considerably with time probably due to the absence of plasmid integration into the rat genome of transduced RPE and choroidal cells. Further studies with inducible or tissue-specific promoter sequences will show whether or not a steady and high expression level of the hFGF2 transgene is sufficient to rescue for a longer period of time the photoreceptor cell layer of transfected RCS eyes. Additional studies are required for assessing the existence of immune reactions which may contribute to the decline of expression of the potentially therapeutic molecules produced by the K8/JTS-1 coated plasmids. The absence of macrophages or microglial infiltrations adjacent or nearby the transduced RPE and choroidal cells and in the overlying rescued neural retinal cells does not eliminate the possibility of immune reactions triggered by the coating peptides.

The present study shows that the JTS-1 / K8 transfection technique can be used for *in vivo* transfection of ocular tissue. The observed local but significant rescue of photoreceptor cells in RCS rats by means of peptide-mediated transfection with the hFGF2 transgene indicates that this protocol could be a promising non-viral somatic gene therapy approach to treat photoreceptor cell dystrophies in patients.

### REFERENCES

AKIMOTO et al., (1999) Ophthalmol. Vis. Sci. **40**, 273-279.
ANCHAN et al. (1991) Neuron **6**, 923-936.
BOK, D. and HALL, M.O. (1971) J. Cell Biol. **49**, 664-682.
BUGLER et al. (1991) Mol. Cell. Biol. 11, 573-577.
CAO et al (1997) Invest. Ophthalmol. Vis. Sci. **38**, 1358-1365.
CAYOUETTE, M. and GRAVEL C. (1997) Hum. Gene Ther. **8**, 423-430.
FAKTOROVICH et al (1990) Nature **347**, 83-86.
FAKTOROVICH et al (1992) J. Neurosci. **12,** 3554-3567.
FERNIG, D.G. and GALLAGHER, J.T. (1994) Prog. Growth Factor Res. **5,** 353-377.
GAJDUSEK, C.M. and CARBON, S. (1989) J. Cell. Physiol. **139,** 570-579.
GAUR et al. (1992) Exp. Eye Res. **54**, 91-101.
GOTTSCHALK et al. (1996) Gene Therapy **3**, 448-457.
GOUREAU et al. (1993a) Neuroreport **5**, 233-236.
GOUREAU et al. (1993b) Proc. Natl. Acad. Sci. USA **90**, 4276-4280.
GROGER et al. (1989) (erratum; Gene (1989) **83**, 395). Gene **81**, 285-294.
GUILLEMOT, F. and CEPKO, C.L. (1992) Development **114**, 743-754.
GUILLONNEAU et al. (1997) Exp. Cell Res. **233**, 198-206.
JACKSON et al. (1992) Proc. Natl. Acad. Sci. USA **89**, 10691-10695.
JIAO et al. (1992) Exp. Neurol. **115,** 400-413.
KAHN et al. (1977) Am. J. Epidemiol. **106**, 17-32.
KARSAN et al. (1997) Am. J. Pathol. **151,** 1775-1784.
KLAGSBRUN, M. (1991) a Keystone sympo., Keystone, CO, USA. New Biol. **3**, 745-749.
KLEIN, B.E. and KLEIN, R. (1982) Arch. Ophthalmol. **100**, 571-573.
LAVAIL et al. (1975) J. Hered. **66**, 242-244.
LAVAIL, M.M. (1981a) Friedenwald lecture. Invest. Ophthalmol. Visual Sci. **21**, 638-657.
LAVAIL, M.M. (1981b) J. Hered. **72**, 294-296.
LEI et al. (1996) Gene Therapy **3**, 427-436.
LI, L. and TURNER, J.E. (1988a) Exp. Eye Res. **47**, 771-785.
LI, L. and TURNER, J.E. (1988b) Exp. Eye Res. **47,** 911-917.
LI et al. (1991) Curr. Eye Res. **10,** 947-958.
LI, T. and DAVIDSON, B.L. (1995) Proc. Natl. Acad. Sci. USA **92,** 7700-7704.
LUFT, J.H. (1961) J. Biophys. Biochem. Cytol. **9**, 409.
MALECAZE et al. (1993) J. Cell. Physiol. **154,** 631-642.
MARTINEZ et al. (1982) Am. J. Ophthalmol. 94, 181-189.
MCLAREN, M.J. and INANA, G. (1997) FEBS Letters **412,** 21-29.
MIGNATTI, P. and RIFKIN, D.B. (1991) J. Cell Biochem. **47,** 201-207.
MIGNATTI et al. (1992) J. Cell. Physiol. **151**, 81-93.
MULLEN, R.J. and LAVAIL, M.M. (1976) Science **192,** 799-801.
PERRY et al. (1995) Curr. Eye Res. **14**, 585-592.
PRATS et al. (1989) Proc. Natl. Acad. Sci. USA **86**, 1836-1840.
QUARTO et al. (1991) J. Cell Physiol. **147,** 311-318.
RAKOCZY et al. (1993) Invest. Ophthalmol. Vis. Sci. **34**, 1845-1852.
RIBEAUDEAU et al. (1997) Acad. Sci. Paris, Science de la vie / Life Sciences **320**, 523-532.
ROGELJ et al. (1989) J. Cell. Biochem. **39**, 13-23.
SHEEDLO et al. (1989) Exp. Eye Res. **48**, 841-854.
SHEEDLO et al. (1993) Exp. Eye Res. **57**, 753-761.
SHEEDLO, H.J. and TURNER, J.E. (1996) Develop. Brain Res. **93**, 88-99.
STEELE et al. (1993) Proc. Natl. Acad. Sci. USA **90**, 1526-1530.
TSO et al. (1994) Ophthalmol. Vis. Sci. **35**, 2693-2699.
TURNER, D.L. and CEPKO, C.L. (1987) Nature **328,** 131-136.
TURNER et al. (1990) Neuron **4**, 833-845.
UTEZA et al. (1999). Proc. Natl. Acad. Sci. USA. **96**, 3126-3131.
WEN et al. (1995) J. Neurosci. **15,** 7377-7385.
WOHLGEMUTH et al. (1996) Gene Therapy **3**, 503-512.
YATES et al. (1985) Nature **313**, 812-815.
ZOLOTUKHIN et al. (1996) J. Virol. **70**, 4646-4654.

**Table 1 :**

| Analysis of photoreceptor cell rescue from transfected eyes | | |
|---|---|---|
| **Experimental treatments** | **average % (+/- s.e.m.) field points with delayed cell dystrophy** | |
| | **Cryosection study** (animal group II) | **Epon study** (animal group III) |
| **I** pREP-hFGF2 injection (n=6) | 14.50 (1.84) | 17.5 (2.2) |
| **II** Non-injected control eyes (contralateral to hFGF2 transfected eyes) (n=6) | 2.65 (0.64) *** | - |
| **III** vehicle injection (n=6) | 3.98 (0.71) ** | - |
| **IV** Non-injected control eyes (contralateral to vehicle injected eyes) (n=6) | 4.05 (0.60) | - |
| **V** PREP5β injection (n=5) | - | 2.6 (1.2) ooo |
| **VI** Non-injected control eyes (n=6) | - | 1.3 (0.5) oo |

| | | |
|---|---|---|
| The field of delayed cell degeneration was determined on 2-dimensional representations of the retina. It corresponds to the area outlined by field points which fulfill the criterion for delayed cell degeneration. Cryosection study : two-tailed paired Student's t-test; *** P=0.001 (I vs. II), | | |
| ** P=0.002 (I vs. III). Epon study : two-tailed unpaired Student's t-test | | |
| ^{ooo} P=0.001 (I vs. II), | | |
| ^{oo} P=0.002 (I vs. III). | | |

## Claims

1. The use of a cationic peptide and a fusogenic peptide in the preparation of a composition for delivering a polynucleotide to an ocular cell in vitro, ex vivo or in vivo.

2. Use according to claim 1, wherein the cationic peptide comprises 3 to 60 amino acid residues, preferably 5 to 40, more preferably 5 to 20.

3. Use according to claim 2, wherein the cationic peptide comprises a stretch of at least five, preferably between 5 and 15 positively charged amino acid residues.

4. Use according to any one of the preceding claims, wherein the fusogenic peptide comprises 3 to 60 amino acid residues, preferably 5 to 40, more preferably 10 to 30.

5. Use according to any one of the preceding claims, wherein the polynucleotide encodes a biologically active product, more preferably a biologically active polypeptide.

6. Use according to claim 5, wherein the polynucleotide is a double-stranded, circular DNA, such as a plasmid.

7. Use according to any one of the preceding claims, of a bifunctional peptide comprising a cationic moiety and a fusogenic moiety.

8. Use according to any one of claims 1 to 7, wherein the overall ratio (R) of the positive charges of the peptide(s) on the negative charges of the polynucleotide (+/-) ranges from 0.1 to 5, preferably from 0.5 to 1.5, for in vivo uses.

9. Use according to any one of claims 1 to 7, wherein the overall ratio (R) of the positive charges of the peptide(s) on the negative charges of the polynucleotide (+/-) ranges from 0.1 to 15 for in vitro or ex vivo uses.

10. Use according to any one of the preceding claims for delivering a polynucleotide to retinal cells.

11. Use according to claim 10 for delivering a polynucleotide to retinal pigment epithelial cells.

12. Use according to claim 10 for delivering a polynucleotide to choroidal cell.

13. Use according to any one of the preceding claims for treating photoreceptor cell dystrophies.

14. Use according to claim 13 for delaying photoreceptor cell degeneration.

15. A method of delivering a polynucleotide to an ocular cell in vitro or ex vivo comprising contacting said ocular cell with a composition comprising said polynucleotide complexed with a cationic peptide and a fusogenic peptide.

16. A method of delivering a polynucleotide to an ocular cell in vitro or ex vivo comprising:
(c) contacting said polynucleotide with a cationic peptide and a fusogenic peptide to form a complex between said polynucleotide and said peptides, and
(d) contacting the ocular cell with said complex.


SEQUENCE LISTING

<110> Association Retina France
      Universite Rene Descartes (Paris V)

<120> Peptide-mediated ocular cell transfection

<130> B0041EP

<140>
<141>

<160> 7

<170> PatentIn Ver. 2.1

<210> 1
<211> 13
<212> PRT
<213> Artificial Sequence

<220>
<223> Description of Artificial Sequence: Peptide

<400> 1
Tyr Lys Ala Lys Lys Lys Lys Lys Lys Lys Lys Trp Lys
  1               5                  10

<210> 2
<211> 20
<212> PRT
<213> Artificial Sequence

<220>
<223> Description of Artificial Sequence: Peptide

<400> 2
Gly Leu Phe Glu Ala Leu Leu Glu Leu Leu Glu Ser Leu Trp Glu Leu
  1               5                  10                  15

Leu Leu Glu Ala
             20

<210> 3
<211> 7
<212> PRT

<213> Artificial Sequence

<220>
<223> Description of Artificial Sequence: Peptide

<400> 3
Pro Lys Lys Lys Arg Lys Val
 1               5



<210> 4
<211> 15
<212> PRT
<213> Artificial Sequence

<220>
<223> Description of Artificial Sequence: Peptide

<400> 4
Lys Arg Pro Ala Ala Thr Lys Lys Ala Gly Gln Ala Lys Lys Lys
 1               5                  10                  15



<210> 5
<211> 30
<212> PRT
<213> Artificial Sequence

<220>
<223> Description of Artificial Sequence: Peptide

<400> 5
Trp Glu Ala Lys Leu Ala Lys Ala Leu Ala Lys Ala Leu Ala Lys His
 1               5                  10                  15

Leu Ala Lys Ala Leu Ala Lys Ala Leu Lys Ala Cys Glu Ala
            20                  25                  30



<210> 6
<211> 60
<212> DNA
<213> Artificial Sequence

<220>
<223> Description of Artificial Sequence:
      oligonucleotide

<400> 6
aaaaccatgg cttacccata cgatgttcca gattacgcta gcgcagccgg gagcatcacc 60

<210> 7
<211> 23
<212> DNA
<213> Artificial Sequence

<220>
<223> Description of Artificial Sequence:
      oligonucleotide

<400> 7
gaccaattat ccaaactgag ctt                                          23